# EUROPEAN PATENT SPECIFICATION

(11) **EP 1 249 239 B1**
(45) Date of publication and mention of the grant of the patent: **21.05.2008**
(21) Application number: 00981741.2
(22) Date of filing: 15.12.2000
(51) Int. Cl.: A61K 31/60, A61P 17/04, A61K 9/06, A61K 9/70

(54) **ANTIPRURITIC AGENTS FOR EXTERNAL USE**
WIRKSTOFFE GEGEN PRURITUS ZUM ÄUSSERLICHEN GEBRAUCH
AGENT ANTIPRURITIQUE A USAGE EXTERNE

(30) Priority: 28.12.1999 JP 37354799
(43) Date of publication of application: 16.10.2002
(73) Proprietor: TEIKOKU SEIYAKU CO., LTD., Okawa-gun, Kagawa 769-2695 (JP)
(72) Inventor: INAMOTO, Yukiko, Kagawa-gun, Kagawa 761-1703 (JP); KAWATA, Mitsuhiro, Okawa-gun, Kagawa 769-2702 (JP); KAWABATA, Seiichiro, Okawa-gun, Kagawa 769-2601 (JP); NAKAYAMA, Daisuke, Takamatsu-shi, Kagawa 761-0104 (JP); HISAICHI, Shin-ichi, Kita-gun, Kagawa 761-0612 (JP); TOKUDA, Masaaki, Takamatsu-shi, Kagawa 761-8071 (JP)
(74) Representative: Albrecht, Thomas
(86) International application number: PCT/JP2000/008888
(87) International publication number: WO 2001/047525

(56) References cited:
- JP-A- 1 029 315
- US-A- 4 975 269
- US-A- 5 763 425
- US-A- 5 932 230
- FJELLNER B ET AL: "PRURITUS IN POLYCYTHEMIA VERA: TREATMENT WITH ASPIRIN AND POSSIBILITY OF PLATELET INVOLVEMENT" ACTA DERMATO-VENEREOLOGICA, vol. 59, no. 6, 1979, pages 505-512, XP000608616 ISSN: 0001-5555
- JUNGNICKEL P.W. ET AL.: 'Effect of two aspirin pretreatment regimens on niacin-induced catuneous reactions' JOURNAL OF GENERAL INTERNAL MEDICINE vol. 12, no. 10, 1997, pages 591 - 596, XP002956938
- YOSIPOVITCH G. ET AL.: 'Topically applied aspirin rapidly decreases histamine-induced itch' ACTA DERM.-VENEREOL. vol. 77, no. 1, 1997, pages 46 - 48, XP002956939
- HAGERMARK OSTEN: 'Influence of antihistamines, sedatives and aspirin on experimental itch' ACTA DERM.-VENEREOL. vol. 53, no. 5, 1973, pages 363 - 368, XP002956940
- DALEY B.M. ET AL.: 'Effect of aspirin on pruritus' BRITISH MEDICAL JOURNAL vol. 293, no. 6552, 1986, page 907, XP002956941

## Description

### Technical Field

The present invention relates to the use of acetylsalicylic acid for the manufacture of external preparations having an excellent antipruritic activity.

### Background Art

Recently according to change of life style, diseases with strong itching, such as atopic dermatitis, urticaria, skin pruritus, etc. have rapidly increased. Further, sting by insects (bite) often elicits very strong itching.

Nowadays many antipruritic agents such as antihistamines etc. are sold. In case of an oral preparation thereof being taken, it is anxious for its side effects, such as sleepiness, laziness, etc.

On the other hand an antipruritic activity of an external preparation containing an antihistamine or a nonsteroidal antiinflammatory agent is not satisfactory, and especially the preparation containing an antihistamine is also anxious for its side effects such as dermal anaphylaxis, and the preparation containing a nonsteroidal antiinflammatory agent is also anxious for its side effects, such as dermal irritation, contact dermatitis, etc.

Furthermore, although steroids for an external application which are essential for the therapy of atopic dermatitis, are very useful for eczema, skin pruritus, sting by insects, etc., these steroids are not only anxious for their side effects, such as atrophia cutis, steroid flush, angiotelectasis, etc., when repeatedly taken, but also these steroids are transdermally absorbed to migrate to blood and have a possibility to give systemically bad effects.

Acetylsalicylic acid (Hereinafter it may be written as Aspirin.) has a strong analgesic activity, an antifebrile activity and an antirheumatic activity being less in its side effects and being superior in its safety. Therefore, Aspirin has been widely used from of old.

Recently there have been the studies for applications of external preparations containing acetylsalicylic acid. As a result a composition being superior in transdermal absorption, a new gel-preparation, a tape preparation and a plaster are disclosed in published patent specifications, etc.

Furthermore, as a new use of acetylsalicylic acid in form of an external preparation, ointments for treating neuralgia (Japanese Patent Pub. A3-72426), external preparations for treating skin injury (Japanese Patent Pub. A9-235232), a transdermal administration system for treatment of thrombosis and for prophylactic treatment of cancer (Japanese Patent Pub. Tokuhyo 8-504198) are illustrated.

US 4,975,269 discloses a shelf stable solution of aspirin, free of moieties reactive with aspirin, suitable for topical application to the skin. Moreover, US 5,763,425 discloses the use of acetylsalicylic acid as an antithrombotic agent and as an agent to treat other medical conditions benefiting from suppression of thromboxane levels. G. Yosipovitch et al., "Topically Applied Aspirin Rapidly Decreases Histamine-induced Itch", Acta Derm Venereol, 77, 1997, pp. 46-48 discloses a study in which the effect of topical aspirin and its model vehicle dichloromethane on itch induced with histamine in 16 subjects has been tested.

### Disclosure of Invention

The present invention is to provide external preparations which have an excellent antipruritic activity and are less in their side effects.

The present inventors have earnestly studied and as a result, have found that an external preparation containing acetylsalicylic acid as an active ingredient is less in its side effects and shows an excellent antipruritic activity. Thus the present invention has been completed.

Namely, the present invention provides the use of acetylsalicylic acid for the preparation of an ointment, suspension, emulsion, lotion, cataplasm, tape, aerosol or external powder for the treatment of itching caused by skin diseases selected from the group consisting of atopic dermatitis, eczema, contact dermatitis, seborric dermatitis, urticaria and puerile strophulus; sting by insects; dermal pruritus; senile pruritus; skin injury selected from the group consisting of a cut, wound after operation and burn; and metabolic diseases selected from the group consisting of hepatocirrhosis, uremia, chronic nephritis and endocrine or dishormonic disease.

Acetylsalicylic acid contained in the external preparation of the present invention is described in the Pharmacopoeia of Japan XIII.

The amount of acetylsalicylic acid in the external preparation depends on form of the preparation, but is 0.05-80%, preferably 0.05-70%, more preferably 0.1-50% per total amount by weight. When the amount of acetylsalicylic acid is more than 80% by weight, it is impossible to maintain the physical property as an external preparation. When the amount of acetylsalicylic acid is less than 0.05% by weight, the antipruritic activity by acetylsalicylic acid does not show enough. The amount as more than 80% or less than 0.05% by weight, therefore is not preferable.

The diseases with itching for which the external preparation of the present invention is used are itching with skin diseases, namely atopic dermatitis, eczema, contact dermatitis, seborric dermatitis, urticaria, puerile strophulus, sting by insects, dermal pruritus; senile pruritis; itching with metabolic diseases, namely hepatocirrhosis, uremia and chronic nephritis; itching with endocrine or dyshormonic disease such as diabetis; and itching with skin injury, namely a cut, wound after operation, or burn.

The external preparation of the present invention is a preparation in which acetylsalicylic acid can be directly applied on the local surface of skin. Such preparations are ointments, suspensions, emulsions, lotions , cataplasms, tapes, aerosols and external powders (powders for external use).

As other ingredients of the preparation of the present invention can be used any ingredient used in the ordinarily external preparation.

In case of ointments, creams, gels and lotions, bases, such as white vaseline (petrolatum), yellow vaseline, lanolin, purified bee wax, cetanol, stearyl alcohol, stearic acid, hydrogenated oil, hydrocarbon gel, polyethylene glycol, liquid paraffin and squalane; solvents or solubilizing agents, such as oleic acid, isopropyl myristate, glycerol triisooctanoate, crotamiton, diethyl sebacate, diisopropyl sebacate, diisopropyl adipate, hexyl laulate, a fatty acid, a fatty acid ester, an aliphatic alcohol, and a plant oil; antioxidants, such as a tocopherol derivative, L-ascorbic acid, dibutylhydroxytoluene and butylhydroxyanisole; antiseptics such as p-hydroxybenzoate; humectants, such as glycerin, propylene glycol and sodium hyaluronate; surfactants, such as a polyoxyethylene derivative, a glycerol fatty acid ester, a sucrose fatty acid ester, a sorbitan fatty acid ester, a propylene glycol fatty acid ester and lecithin; thickening agents, such as carboxyvinyl polymer, xanthan gum, carboxymethyl cellulose, sodium carboxymethyl cellulose, hydroxypropyl cellulose and hydroxypropylmethyl cellulose; stabilizers; preservatives; absorption promoters; and other suitable fillers may be added.

In case of cataplasms, tackifiers, such as polyacrylic acid and polyacrylic acid copolymer; crosslinkers, such as aluminum sulfate, aluminum potassium sulfate, aluminum chloride, magnesium aluminometasilicate and dihydroxyalminum aminoacetate; thickening agents, such as sodium polyacrylate, polyvinyl alcohol, polyvinylpyrrolidone, gelatin, sodium alginate, carboxymethyl cellulose, sodium carboxymethyl cellulose, hydroxypropyl cellulose and hydroxypropylmethyl cellulose; polyhydric alcohols, such as glycerin, polyethylene glycol (macrogol), propylene glycol and 1,3-butanediol; surfactants such as a polyoxyethylene derivative; perfumes such as ℓ-menthol; antiseptics such as p-hydroxybenzoate; purified water; and other suitable fillers may be added.

In case of tapes, tacking agents, such as a stylene-isoprene-stylene block copolymer and an acrylate resin; tackifier resins, such as an alicyclic saturated hydrocarbon resin, a hydrogenated rosin resin and a terpene resin; softeners, such as liquid gum and liquid paraffin; antioxidants such as dibutylhydroxytoluene; polyhydric alcohols such as polyethylene glycol; absorption promoters such as oleic acid; surfactants such as a polyoxyethylene derivative; and other suitable fillers may be added. In addition a water-absorbable polymer, such as sodium polyacrylate and polyvinyl alcohol, and a small amount of purified water may be added to prepare tape preparations containing water.

In case of aerosols, bases, such as white vaseline (petrolatum), yellow vaseline, lanolin, purified bee wax, cetanol, stearyl alcohol, stearic acid, hydrogenated oil, hydrocarbon gel, polyethylene glycol, liquid paraffin and squalane; solvents or solubilizing agents, such as oleic acid, isopropyl myristate, isopropyl adipate, diisopropyl sebacate, glycerol triisooctanoate, crotamiton, diethyl sebacate, hexyl laurate, a fatty acid, a fatty acid ester, an aliphatic alcohol and a plant oil; antioxidants, such as a tocopherol derivative, L-ascorbic acid, dibutylhydroxytoluene and butylhydroxyanisole; antiseptics such as p-hydroxybenzoate; humectants, such as glycerin, propylene glycol and sodium hyaluronate; surfactants, such as a polyoxyethylene derivative, a glycerol fatty acid ester, a sucrose fatty acid ester, a sorbitan fatty acid ester, a propylene glycol fatty acid ester and lecithin; thickening agents, such as carboxyvinyl polymer, xanthan gum, carboxymethyl cellulose, sodium carboxymethyl cellulose, hydroxypropyl cellulose and hydroxypropylmethyl cellulose, as used in the ointments, the creams, the gels, the suspensions, the emulsifying agents or the lotions; stabilizers; buffering agents; sweetening agents; suspending agents; emulsifying agents; flavors; preservatives; solubilizing agents; and other suitable fillers, may be added.

In case of external powders, fillers, such as potato starch, rice starch, corn starch, talc and zinc oxide, and other suitable additives may be added to them.

The external preparation of the present invention can be prepared, for example by well kneading each ingredient, if necessary with a suitable base, in accordance with a usual manner to prepare external preparations.

The amount of acetylsalicylic acid as an active ingredient depends on the preparation, but is 0.05-30% by weight in ointments, creams, gels and lotions, is 0.1-20% by weight in cataplasms, is 5-50% by weight in tapes, and is 10-80% by weight in external powders.

Thus prepared preparation is applied to the lesion, if necessary.

### Best Mode for Carrying out Invention

The external preparations containing acetylsalicylic acid of the present invention are explained by examples and experimental examples.

### Examples 1-10 (Ointments)

According to ingredients indicated in Table 1, hydrocarbon gel and a solvent (oleic acid, Tween 80, crotamiton, diisopropyl adipate or isopropyl myristate) were dissolved by warming on a water bath, and thereto was added acetylsalicylic acid (Aspirin) to dissolve or well disperse under stirring. Then the mixture was cooled under stirring to prepare ointments.

**Table 1: Ingredients of ointments containing Aspirin**

| Examples | 1 | 2 | 3 | 4 | 5 | 6 | 7 | 8 | 9 | 10 |
|---|---|---|---|---|---|---|---|---|---|---|
| Ingredients | Ingredient ratio (wt%) | | | | | | | | | |
| Aspirin | 0.1 | 0.5 | 2.0 | 10.0 | 20.0 | 2.0 | 2.0 | 2.0 | 2.0 | 2.0 |
| Oleic acid | - | - | - | - | - | 5.0 | - | - | - | - |
| Tween 80 | - | - | - | - | - | - | 5.0 | - | - | - |
| Crotamiton | - | - | - | - | - | - | - | 5.0 | - | - |
| Diisopropyl adipate | - | - | - | - | - | - | - | - | 5.0 | - |
| Isopropyl myristate | 2.5 | 2.5 | 2.5 | 2.5 | 2.5 | - | - | - | - | 5.0 |
| Hydrocarbon gel | 97.4 | 97.0 | 95.5 | 87.5 | 77.5 | 93.0 | 93.0 | 93.0 | 93.0 | 93.0 |

### Examples 11-15 (Lotions)

According to ingredients indicated in Table 2, Aspirin was added to a warmed oil layer to dissolve or disperse. Separately other ingredients were dissolved in previously warmed purified water, and the oil layer was added thereto under vigorously stirring. The mixture was been mixing to homogeneity under gradually cooling to prepare lotions.

**Table 2: Ingredients of lotions containing Aspirin**

| | | | | | |
|---|---|---|---|---|---|
| Examples | 11 | 12 | 13 | 14 | 15 |
| Ingredients | Ingredient ratio (wt%) | | | | |
| Aspirin | 0.5 | 2.0 | 10.0 | 5.0 | 5.0 |
| Crotamiton | 1.0 | 2.0 | 5.0 | - | - |
| Isopropanol | - | - | - | 2.0 | - |
| Diisopropyl sebacate | - | - | - | - | 5.0 |
| Squalane | 3.0 | 3.0 | 3.0 | 3.0 | 3.0 |
| Cetanol | 3.0 | 3.0 | 3.0 | 3.0 | 3.0 |
| Solbitan sesquioleate | 0.5 | 0.5 | 0.5 | 0.5 | 0.5 |
| Polyoxy(20)cetyl ether | 1.5 | 1.5 | 1.5 | 1.5 | 1.5 |
| Propylene glycol | 5.0 | 5.0 | 5.0 | 5.0 | 5.0 |
| Triethanolamine | 0.4 | 0.4 | 0.4 | 0.4 | 0.4 |
| Purified water | 85.1 | 82.6 | 71.6 | 79.6 | 76.6 |

### Examples 16-20 (Gels)

According to ingredients indicated in Table 3, after a water soluble polymer was dissolved on a water bath, Aspirin was dissolved or dispersed in a solvent and these ingredients with other bases were being mixed to homogeneity to prepare gels.

**Table 3: Ingredients of gels containing Aspirin**

| Examples | 16 | 17 | 18 | 19 | 20 |
|---|---|---|---|---|---|
| Ingredients | Ingredient ratio (wt%) | | | | |
| Aspirin | 0.1 | 2.0 | 10.0 | 5.0 | 5.0 |
| Crotamiton | 5.0 | 5.0 | 5.0 | 3.0 | - |
| Isopropanol | - | - | - | 3.0 | 5.0 |
| Propylene glycol | 45.0 | 45.0 | 45.0 | 45.0 | 45.0 |
| Polyacrylic acid | 25.0 | 25.0 | 25.0 | 25.0 | 25.0 |
| Triethanolamine | 0.7 | 0.7 | 0.7 | 0.7 | 0.7 |
| Purified water | 24.2 | 22.3 | 14.3 | 18.3 | 19.3 |

### Examples 21-25 (Creams)

According to ingredients indicated in Table 4, after a solid base was dissolved on a water bath, Aspirin dissolved or dispersed in a solvent was added thereto. A watersoluble base was dissolved in water and its warmed solution was added to the mixture. The mixture was kneaded until it became homogenous to prepare creams.

**Table 4: Ingredients of ointments containing Aspirin**

| Examples | 21 | 22 | 23 | 24 | 25 |
|---|---|---|---|---|---|
| Ingredients | Ingredient ratio (wt%) | | | | |
| Aspirin | 0.5 | 2.0 | 10.0 | 2.0 | 2.0 |
| Crotamiton | 2.5 | 2.5 | 2.5 | 5.0 | - |
| Sesame oil | - | - | - | - | 5.0 |
| Diisopropyl sebacate | 2.5 | 2.5 | 2.5 | - | - |
| Cetanol | 9.0 | 9.0 | 9.0 | 9.0 | 9.0 |
| White vaseline | 8.0 | 8.0 | 8.0 | 8.0 | 8.0 |
| Hexyldecanol | 1.0 | 1.0 | 1.0 | 1.0 | 1.0 |
| Polyethylene glycol monostearate | 2.0 | 2.0 | 2.0 | 2.0 | 2.0 |
| Polyoxy(9) lauryl ether | 2.8 | 2.8 | 2.8 | 2.8 | 2.8 |
| Polyoxy(23) cetyl ether | 2.0 | 2.0 | 2.0 | 2.0 | 2.0 |
| Propylene glycol | 12.0 | 12.0 | 12.0 | 12.0 | 12.0 |
| Methylparaben | 0.1 | 0.1 | 0.1 | 0.1 | 0.1 |
| Propylparaben | 0.1 | 0.1 | 0.1 | 0.1 | 0.1 |
| Purified water | 57.5 | 56.0 | 48.0 | 56.0 | 56.0 |

### Examples 26-30 (Tapes)

According to ingredients indicated in Table 5, to a tacking agent consisting of an acrylate resin or a stylene-isoprene-stylene block copolymer were added an alicyclic saturated hydrocarbon resin, liquid paraffin, polybutene, an antioxidant, etc. and the mixture was dissolved in an organic solvent such as toluene etc. under stirring, or the mixture was melted by heating under stirring. Thereto was added Aspirin and the resulting mixture was spread on releasing paper and in case of a solution type, was spread on releasing paper and dried. The releasing paper was laminated on a flexible support to be cut into a desired size to prepare tapes.

**Table 5: Ingredients of tapes containing Aspirin**

| Examples | 26 | 27 | 28 | 29 | 30 |
|---|---|---|---|---|---|
| Ingredients | Ingredient ratio (wt%) | | | | |
| Aspirin | 10.0 | 30.0 | 50.0 | 30.0 | 30.0 |
| Isopropyl myristate | - | - | - | - | 5.0 |
| Diisopropyl adipate | - | - | - | 5.0 | - |
| Crotamiton | 5.0 | 5.0 | 7.0 | - | - |
| Acrylate-vinyl acetate copolymer | - | - | - | - | 65.0 |
| Stylene-isoprene-stylene block copolymer | 20.0 | 13.4 | 7.5 | 13.4 | - |
| Alicyclic saturated hydrocarbon resin | 42.0 | 23.5 | 11.7 | 23.5 | - |
| Polybutene | 15.0 | 11.6 | 5.6 | 11.6 | - |
| Liquid paraffin | 7.0 | 15.5 | 17.2 | 15.5 | - |
| Dibutyl hydroxytoluene | 1.0 | 1.0 | 1.0 | 1.0 | - |

### Examples 31-33 (Cataplasms)

According to ingredients indicated in Table 6, a tackifier such as a polyacrylic acid etc. and a thikening agents were dissolved under heating in a polyhydric alcohol such as glycerin etc. After cooling, Aspirin and other fillers were blended to homogeneity and thereto was added a crosslinker to prepare an adhesive gel base. The gel base was spread on a suitable support such as a non-woven fabric to be cut in a desired size to prepare cataplasms.

**Table 6: Ingredients of cataplasms containing Aspirin**

| Examples | 31 | 32 | 33 |
|---|---|---|---|
| Ingredients | Ingredient ratio (wt%) | | |
| Aspirin | 0.5 | 2.0 | 10.0 |
| Polyacrylic acid | 8.0 | 8.0 | 8.0 |
| Sodium polyacrylate | 4.0 | 4.0 | 4.0 |
| Sodium carboxy cellulose | 5.0 | 5.0 | 5.0 |
| Tartaric acid | 1.6 | 1.6 | 1.6 |
| Dihydroxyalminum aminoacetate | 0.07 | 0.07 | 0.07 |
| Glycerin | 34.5 | 33.0 | 25.0 |
| Crotamiton | 2.0 | 2.0 | 2.0 |
| Sesame oil | 1.0 | 1.0 | 1.0 |
| Purified water | 43.33 | 43.33 | 43.33 |

### Examples 34-36 (Powders)

According to ingredients indicated in Table 7, potato starch, zinc oxide and Aspirin were well mixed to prepare powders.

**Table 7: Ingredients of powders containing Aspirin**

| Examples | 34 | 35 | 36 |
|---|---|---|---|
| Ingredients | Ingredient ratio (wt%) | | |
| Aspirin | 20.0 | 40.0 | 80.0 |
| Potato starch | 76.0 | 56.0 | 16.0 |
| Zinc oxide | 4.0 | 4.0 | 4.0 |

### Comparative examples 1-2

According to the method of preparing ointments, ointments having ingredients of Table 8 (comparative examples 1-2) were prepared.

**Table 8: Ingredients of ointments (Comparative examples)**

| Comparative examples | 1 | 2 |
|---|---|---|
| Ingredients | Ingredient ratio (wt%) | |
| Diphenhydramine | 1.0 | - |
| Dexamethasone | - | 0.1 |
| Propylene glycol | 10.0 | 10.0 |
| Isopropyl myristate | 5.0 | 5.0 |
| Hydrocarbon gel | 84.0 | 84.9 |

Test [A]: An antipruritic activity was tested by administering the external preparation of the present invention for treating pruritus to patients (volunteers).

The degree of itching-improvement was evaluated based on the following five steps standard:
A: Remarkably effective,
B: Effective,
C: Slightly effective,
D: No change,
E: Worse.

Being slightly effective (C) or more than slightly effective (A, B), degree was judged to be effective, and its effective rate was calculated.

In the following experiments 1-4, an ointment containing diphenhydramine having antihistaminic activity (comparative example 1) and an ointment containing dexamethasone (steroid) of comparative example 2 were evaluated, too.

In experiment 5, an ointment containing isopropyl azulene (0.033%) and purified lanolin and white vaseline as bases, which was commercially available as a therapeutic agent for inflammatic dermatitis (comparative example 3), was used as a comparative example.

### Experiment 1: Improvement of itching due to sting by insects on patients

The external preparations containing Aspirin and the controls were applied to lesions on each patient (total 45 volunteers) with itching due to sting by insects and the degree of improvement of itching was evaluated.

The result is shown in Table 9.

**Table 9: Degree of improvement of itching due to sting by insects on patients**

| Groups | Drugs (wt%) | No. of patient | Evaluation | | | | | Effective rate(%) |
|---|---|---|---|---|---|---|---|---|
| | | | A | B | C | D | E | |
| Ointment base | - | 5 | 0 | 0 | 0 | 4 | 1 | 0 |
| Example | Aspirin | 4 | 0 | 0 | 2 | 2 | 0 | 50.0 |
| | | | | | | | | |
| Example 3 | Aspirin | 7 | 2 | 3 | 1 | 0 | 1 | 85.7 |
| | (2) | | | | | | | |
| Example 4 | Aspirin | 7 | 1 | 3 | 2 | 1 | 0 | 85.7 |
| | (10) | | | | | | | |
| Example 5 | Aspirin | 5 | 2 | 2 | 0 | 1 | 0 | 80.0 |
| | (20) | | | | | | | |
| Example 29 | Aspirin | 7 | 2 | 2 | 2 | 1 | 0 | 85.7 |
| | (30) | | | | | | | |
| Comp. Ex.1 | Diphenhydramine(1) | 5 | 1 | 0 | 1 | 3 | 0 | 40.0 |
| Comp. Ex.2 | Dexamethasone | 5 | 1 | 1 | 1 | 2 | 0 | 60.0 |
| | (0.1) | | | | | | | |

As is clear from the result in Table 9, examples 1, 3-5 and 29 containing Aspirin inhibited itching due to sting by insect and showed the same or superior antipruritic effect, comparing with the ointment base and comparative examples 1 and 2.

### Experiment 2: Degree of improvement of itching due to eczema on patients

The external preparations containing Aspirin and the controls were applied to lesions on each patient (total 32 volunteers) with itching due to eczema and the degree of improvement of itching was evaluated.

The result is shown in Table 10.

**Table 10: Degree of improvement of itching due to eczema on patients**

| Groups | Drugs (wt%) | No. of patient | Evaluation | | | | | Effective rate(%) |
|---|---|---|---|---|---|---|---|---|
| | | | A | B | C | D | E | |
| Ointment base | - | 3 | 0 | 0 | 0 | 2 | 1 | 0 |
| Example 9 | Aspirin | 5 | 1 | 1 | 1 | 2 | 0 | 60.0 |
| | (2) | | | | | | | |
| Example 12 | Aspirin | 6 | 2 | 1 | 2 | 0 | 1 | 83.3 |
| | (2) | | | | | | | |
| Example 17 | Aspirin | 4 | 0 | 1 | 2 | 1 | 0 | 75.0 |
| | (2) | | | | | | | |
| Example 21 | Aspirin | 4 | 1 | 1 | 1 | 0 | 1 | 75.0 |
| | (0.5) | | | | | | | |
| Example 33 | Aspirin | 3 | 0 | 1 | 1 | 1 | 0 | 66.7 |
| | (10) | | | | | | | |
| Comp. Ex. 1 | Diphenhydramine(1) | 3 | 0 | 1 | 0 | 2 | 0 | 33.3 |
| Comp. Ex.2 | Dexamethasone | 4 | 1 | 0 | 1 | 2 | 0 | 50.0 |
| | (0.1) | | | | | | | |

As is clear from the result in Table 10, examples 9, 12, 17, 21 and 33 containing Aspirin more inhibited itching due to eczema and showed a superior antipruritic effect, comparing with the ointment base and comparative examples 1 and 2.

### Experiment 3: Degree of improvement of itching due to dermal pruritus on volunteers

The external preparations containing Aspirin and the controls were applied to lesions on each patient (total 31 volunteers) with itching due to dermal pruritus and the degree of improvement of itching was evaluated.

The result is shown in Table 11.

**Table 11: Degree of improvement of itching due to dermal pruritus on patients**

| Groups | Drugs (wt%) | No. of patient | Evaluation | | | | | Effective rate(%) |
|---|---|---|---|---|---|---|---|---|
| | | | A | B | C | D | E | |
| Ointment base | - | 3 | 0 | 0 | 0 | 2 | 1 | 0 |
| Example 8 | Aspirin | 6 | 0 | 1 | 3 | 1 | 1 | 66.7 |
| | (2) | | | | | | | |
| Example 15 | Aspirin | 4 | 1 | 0 | 2 | 1 | 0 | 75.0 |
| | (2) | | | | | | | |
| Example 20 | Aspirin | 4 | 0 | 1 | 2 | 1 | 0 | 75.0 |
| | (5) | | | | | | | |
| Example 21 | Aspirin | 3 | 0 | 0 | 2 | 1 | 0 | 66.7 |
| | (0.5) | | | | | | | |
| Example 24 | Aspirin | 3 | 0 | 1 | 1 | 1 | 0 | 66.7 |
| | (5) | | | | | | | |
| Comp. Ex. 1 | Diphenhydramine(1) | 4 | 1 | 0 | 1 | 1 | 1 | 50.0 |
| Comp. Ex.2 | Dexamethasone | 4 | 1 | 0 | 1 | 2 | 0 | 50.0 |
| | (0.1) | | | | | | | |

As is clear from the result in Table 11, examples 8, 15, 20, 21 and 24 containing Aspirin more inhibited itching due to dermal pruritus and showed a superior antipruritic effect, comparing with the ointment base and comparative examples 1 and 2.

### Experiment 4: Degree of improvement of itching due to allergic dermatitis on patients

The external preparations containing Aspirin and the controls were applied to lesions on each patient (total 37 volunteers) with itching due to allergic dermatitis and the degree of improvement of itching was evaluated.

The result is shown in Table 12.

**Table 12: Degree of improvement of itching due to allergic dermatitis on patients**

| Groups | Drugs (wt%) | No. of patient | Evaluation | | | | | Effective rate(%) |
|---|---|---|---|---|---|---|---|---|
| | | | A | B | C | D | E | |
| Ointment base | - | 3 | 0 | 0 | 0 | 2 | 1 | 0 |
| Example 10 | Aspirin | 4 | 0 | 1 | 2 | 1 | 0 | 75.0 |
| | (2) | | | | | | | |
| Example 13 | Aspirin | 3 | 0 | 0 | 2 | 0 | 1 | 66.7 |
| | (10) | | | | | | | |
| Example 18 | Aspirin | 3 | 0 | 1 | 1 | 1 | 0 | 66.7 |
| | (10) | | | | | | | |
| Example 25 | Aspirin | 3 | 0 | 0 | 1 | 0 | 1 | 66.7 |
| | (5) | | | | | | | |
| Example 26 | Aspirin | 4 | 1 | 2 | 1 | 1 | 0 | 75.0 |
| | (10) | | | | | | | |
| Example 27 | Aspirin | 4 | 0 | 1 | 0 | 2 | 0 | 50.0 |
| | (30) | | | | | | | |
| Example 28 | Aspirin | 4 | 1 | 2 | 1 | 1 | 0 | 75.0 |
| | (50) | | | | | | | |
| Comp. Ex. 1 | Diphenhydramine(1) | 5 | 0 | 1 | 1 | 2 | 1 | 40.0 |
| Comp. Ex.2 | Dexamethasone | 4 | 0 | 2 | 0 | 2 | 0 | 50.0 |
| | (0.1) | | | | | | | |

As is clear from the result of Table 12, examples 10, 13 and 25-28 containing Aspirin inhibited itching due to allergic dermatitis and showed the same or superior antipruritic effect, comparing with the ointment base and comparative examples 1 and 2.

### Experiment 5: Degree of improvement of itching due to burns on patients

The external preparations containing Aspirin and the controls were applied to lesions on each patient (total 18 volunteers) who complained of itching on the process of treating a burn among patients suffering the burn.

The result is shown in Table 13.

**Table 13: Degree of improvement of itching due to a burn on patients**

| Groups | Drugs (wt%) | No. of patient | Evaluation | | | | | Effective rate(%) |
|---|---|---|---|---|---|---|---|---|
| | | | A | B | C | D | E | |
| Ointment base | - | 4 | 0 | 0 | 0 | 3 | 1 | 0 |
| Example 4 | Aspirin | 4 | 1 | 1 | 1 | 1 | 0 | 75 |
| | (10.0) | | | | | | | |
| Example 9 | Aspirin | 4 | 0 | 2 | 1 | 1 | 0 | 75 |
| | (2.0) | | | | | | | |
| Example 21 | Aspirin | 3 | 1 | 1 | 0 | 1 | 0 | 67 |
| | (0.5) | | | | | | | |
| Comp. Ex.3 | Dimethyl isopropyl azulene | 3 | 0 | 0 | 1 | 3 | 0 | 33 |
| | (0.033) | | | | | | | |

As is clear from the result in Table 13, it was confirmed that examples 4, 9 and 21 containing Aspirin more inhibited itching on the process of treating a burn of the patients, comparing with the ointment base and comparative example 3.

Test [B]: The exacerbation of infectious diseases as one of side effects of steroids has been often problematic. On the other hand decrease of the barrier function of skin is indicated as one of causal factors of allergic dermatitis. As being understood from the fact that a lot of bacteria are present in normal skin tissue, it is well known that when steroids are administered to patients suffered from allergic dermatitis, infectious diseases are apt to be caused due to decrease of immunogenecity.

As such, using examples 2 and 5 of the present invention and comparative examples 1 and 2, the decrease of the immunogenecity was evaluated by setting on the reduction of weight of thymus and adrenal gland as an index.

### Experimental example 6

In this test Wistar male rats (8 weeks old, 6 rats/group) were used. After removal of hairs on the back, the rats were collared not to lick the tested drug (examples 2, 5 and comparative examples 1, 2) on the back. The tested drug (0.5g/rat/day) was applied to the back in the range of 5cm x 5cm for 7 days. After administration the rat was killed and thymus and adrenal gland were extracted from the rat and their weights were measured.

The results are shown in Table 14.

**Table 14: Thymus weight and adrenal gland weight administered by each preparation (per body weight (100g))**

| Groups | Thymus weight (mg) | Adrenal gland weight (mg) |
|---|---|---|
| Non-treated | 159±12 | 20.6±1.0 |
| Ointment base | 160±10 | 19.7±1.3 |
| Example 2 | 160±7 | 21.0±0.7 |
| Example 5 | 162±8 | 20.0±1.3 |
| Comparative example 1 | 158±9 | 18.7±0.7 |
| Comparative example 2 | 37±2 | 15.6±1.0 |

| | | |
|---|---|---|
| Means ± standard error | | |

As shown in Table 14, comparative example 2 much reduced weights of thymus and adrenal gland comparing with examples 2 and 5. The steroid ointment reduced weights of thymus and adrenal gland, one of indexes of immunogenecity, but examples 2 and 5 did not reduce the weights of these organs. Therefore, it is clear that the ointments containing Aspirin of the present invention is less in its side effects and a superior antipruritic agent comparing with the ointment of comparative example 2.

### Possibility of Industrial applicability

The external preparation for treating pruritus of the present invention contains Aspirin as an active ingredient and has an excellent therapeutic effect to itching. Furthermore, the external preparation for treating pruritus of the present invention does not reduce weights of thymus and adrenal gland even by continuous applications and therefore, the preparation of the present invention belongs to the drug showing very little side effects. The present invention can provide the external preparation being not only excellently effective to various itching, but also being very little in its side effects.

## Claims

1. Use of acetylsalicylic acid for the preparation of an ointment, suspension, emulsion, lotion, cataplasm, tape, aerosol or external powder for the treatment of itching caused by skin diseases selected from the group consisting of atopic dermatitis, eczema, contact dermatitis, seborric dermatitis, urticaria and puerile strophulus; sting by insects; dermal pruritus; senile pruritus; skin injury selected from the group consisting of a cut, wound after operation and burn; and metabolic diseases selected from the group consisting of hepatocirrhosis, uremia, chronic nephritis and endocrine or dishormonic disease.

## Patentansprüche

1. Verwendung von Acetylsalicylsäure zur Herstellung einer Salbe, einer Suspension, einer Emulsion, einer Lotion, eines Kataplasmas, eines Pflasters, eines Aerosols oder eines äußerlichen Puders zur Behandlung von Juckreiz, der durch Hauterkrankungen verursacht ist, die ausgewählt sind aus der Gruppe, bestehend aus atopischer Dermatitis, Ekzem, Kontaktdermatitis, Dermatitis seborrhoica, Urtikaria und Strophulus; Insektenstich; Hautpruritus; senilem Pruritus; Hautverletzung, die ausgewählt ist aus der Gruppe, bestehend aus einem Schnitt, einer Wunde nach einer Operation und einer Verbrennung; und metabolischen Erkrankungen, die ausgewählt sind aus der Gruppe, bestehend aus Leberzirrhose, Urämie, chronischer Nephritis sowie endokriner oder dyshormonaler Erkrankung.

## Revendications

1. Utilisation d'acide acétylsalicylique pour la préparation d'une pommade, d'une suspension, d'une émulsion, d'une lotion, d'un cataplasme, d'un ruban, d'un aérosol ou d'une poudre externe pour le traitement des démangeaisons causées par les maladies de peau choisies dans le groupe constitué de la dermite atopique, de l'eczéma, de l'eczéma de contact, de la dermite seborrique, de l'urticaire et du prurigo strophulus; des piqûres d'insectes; du prurit dermique; du prurit sénile; des lésions de la peau choisies dans le groupe constitué d'une coupure, d'une plaie après une opération et d'une brûlure; et des maladies métaboliques choisies dans le groupe constitué de l'hépatocirrhose, de l'urémie, de la néphrite chronique et de maladies endocrine ou hormonales.
